Europäisches Patentamt

European Patent Office

Office européen des brevets

Veröffentlichungsnummer: **0 394 446**
**A1**

# EUROPÄISCHE PATENTANMELDUNG
## veröffentlicht nach Art. 158 Abs. 3 EPÜ

Anmeldenummer: 89901344.5

Anmeldetag: 26.10.88

Internationale Anmeldenummer:
PCT/SU88/00211

Internationale Veröffentlichungsnummer:
WO 90/04435 (03.05.90 90/10)

Int. Cl.⁵: **A61N 5/06**

Veröffentlichungstag der Anmeldung:
**31.10.90 Patentblatt 90/44**

Benannte Vertragsstaaten:
**CH DE FR GB IT LI**

Anmelder: **RADIOTEKHNICHESKY INSTITUT IMENI AKADEMIKA A.L.MINTSA AKADEMII NAUK SSSR**
**ul. 8 Marta, 10-12**
**Moscow, 125083(SU)**

Erfinder: **LOZHENKO, Alexandr Sergeevich**
**Nadsonovsky tupik, 5-32 Moskovskaya obl.**
**Pushkino 141200(SU)**
Erfinder: **SHENDALEV, Viktor Nikolaevich**
**ul. Vucheticha, 4-30**
**Moscow, 125206(SU)**
Erfinder: **DANILOVA, Alla Evgenievna**
**3 Mikhalkovsky per., 7-16**
**Moscow, 125008(SU)**
Erfinder: **RECHITSKY, Vladimir Iliich**
**Peschany per., 8-18**
**Moscow, 125252(SU)**
Erfinder: **NASCHEKIN, Vladimir Konstantinovich**
**Initsiativnaya ul., 2-1-134**
**Moscow, 121357(SU)**

Vertreter: **Görg, Klaus, Dipl.-Ing. et al**
**Hoffmann, Eitle & Partner Arabellastrasse 4**
**(Sternhaus)**
**D-8000 München 81(DE)**

**BIEGSAMER DURCHSICHTIGER KATHETER FÜR PHOTOTHERAPIE BEI ROHRFÖRMIGEN KAVITÄTEN DES ORGANISMUS.**

Biegsamer durchsichtiger Katheter zur Lichtbehandlung der röhrenförmigen Hohlräume von Organismus sieht eine Umhüllung (I) mit ihrem geschlossenen Distalende (2) vor, von dem in einem gewissen Abstand, der in Übereinstimmung mit der jeweiligen Langstreckung des pathologischen Herdes des

röhrenförmigen Hohlraums gewählt ist, innerhalb der Umhüllung (I) und dieser koaxial ein Lichtleiter (3) untergebracht ist, zwischen dessen Stirnflanke (4) und dem geschlossenen Distalende (2) sich ein Hohlraum herausbildet, der mit einem durchsichtigen lichtstreuenden plastischen Stoff (5) mit einem mit der Apertur des Lichtleiters (3) und dem Brechungsfaktor des Werkstoffs der Umhüllung (I) zusammenhängenden Brechungsverhältnis ausgefüllt ist.

BIEGSAMER DURCHSICHTIGER KATHETER ZUR
LICHTBEHANDLUNG DER RÖHRENFÖRMIGEN
HOHLRÄUME DES ORGANISMUS

## Technisches Gebiet

Die vorliegende Erfindung betrifft Vorrichtungen zur Lichtbehandlung der pathologischen Herde eines Organismus der intrakavitären Lokalisation, insbesondere biegsame, durchsichtige Katheter zur Lichtbehandlung der röhrenförmigen Hohlräume des Organismus.

## Zugrundeliegender Stand der Technik

Bereits ist bekannt eine Vorrichtung zur Bestrahlung der schwerzugänglichen Hohlräume des Organismus, in der der Lichtstrom durch ein Linsensystem aus einer Lichtquelle zu dem Lichtleiter hindurchtritt, der aus mehreren Einzelteilen zusammengestellt und biegsam ausgeführt ist (SU, A, 787044).

In der betreffenden Vorrichtung ist der Lichtleiter mit mehreren lichtleitenden Adern und Spiralfedern ausgestattet, wodurch im wesentlichen der Aufbau verwickelt wird und ein kleinerer Wirkungsgrad wegen der grossen Lichtverluste im Lichtleiter mit sich bringt, was letzten Endes eine niedrige Wirksamkeit bei der Ausnutzung des Heilfaktors und demzufolge auch eine Verlängerung der Behandlungsfristen bewirkt.

Bekannt ist auch ein biegsamer, durchsichtiger Katheter zur Lichtbehandlung der röhrenförmigen Hohlräume des Organismus mit geschlossenem Distalende, in einem gewissen Abstand von welchem innerhalb der Katheterumhüllung und mit dieser koaxial ein Lichtleiter hindurchgezogen wird, zwischen dessen Stirnflanke und dem geschlossenen Distalende des Katheters sich ein Hohlraum herausbildet (US, A, 4248214).

In dem betreffenden biegsamen, durchsichtigen Katheter ist der Hohlraum zwischen dem geschlossenen Distalende des Katheters und der Stirnflanke des Lichtleiters mit Luft ausgefüllt, die sich bei der an dem Distalende des Katheters entstehenden Indikatrix der Lichtstrahlungsverteilung in Form einer Kugel auswirkt, wo-

- 2 -

durch keine Gleichmässigkeit und Gleichzeitigkeit der
Belichtung der pathologischen Herde in den Hohlräumen
erreicht werden, die ihrerseits eine niedrige Wirksamkeit der Lichtbehandlung der Hohlräume verursachen.

Falls aber die röhrenförmigen Hohlräume von den genuggrossen Oberflächen beschädigt werden, muss eine Abtastung des beschädigten Feldes mit dem erwähnten Katheter durchgeführt werden, die auch keine Gleichmässigkeit
der Bestrahlung und demzufolge der Wirksamkeit der Behandlung der röhrenförmigen Hohlräume bewirkt.

Offenbarung der Erfindung

Der vorliegenden Erfindung liegt die Aufgabe zugrunde, einen biegsamen, durchsichtigen Katheter zur Lichtbehandlung der röhrenförmigen Hohlräume des Organismus
zu entwickeln, in dem der Hohlraum zwischen dem geschlossenen Distalende des Katheters und der Stirnflanke des
Lichtleiters derart ausgeführt ist, dass die Indikatrix
der Lichtstrahlungsverteilung in Form eines Zylinders
erhalten wird und demzufolge eine wesentliche Steigerung
der Wirksamkeit der Lichtbehandlung der röhrenförmigen
Hohlräume des Organismus erreicht wird.

Dies wird dadurch gelöst, dass in dem biegsamen
durchsichtigem Katheter zur Lichtbehandlung der röhrenförmigen Hohlräume des Organismus mit seinem geschlossenen Distalende, von dem in einem Abstand innerhalb der
Katheterumhüllung mit dieser koaxial ein Lichtleiter
untergebracht ist, zwischen dessen Stirnflanke und dem
geschlossenen Distalende des Katheters sich ein Hohlraum
herausbildet, erfindungsgemäss ein durchsichtiger, lichtstreuender, plastischer Stoff mit dem Brechungsverhältnis n ausgenutzt wird, das sich aus der Beziehung

$$n \geq \sqrt{A^2 + n_I^2}$$

ergibt, worin
A die Apertur des Lichtleiters und $n_I$ das Brechungsverhältnis der Katheterumhüllung bedeuten, wobei der genannte Plaststoff innerhalb des Hohlraums zwischen dem
geschlossenen Distalende des Katheters und der Stirn-

- 3 -

flanke des Lichtleiters untergebracht ist, wobei der Abstand zwischen diesen mit der Streckeinlänge eines pathologischen Herdes des jeweiligen Hohlraums übereinstimmt.

Durch eine derartige Ausführung des erfindungsgemässen biegsamen durchsichtligen Katheters zur Lichtbehandlung der röhrenförmigen Hohlräume des Organismus wird eine Gleichmässigkeit der Belichtung der langgestreckten Beschädigungen in den röhrenförmigen Hohlräumen des Organismus ohne zusätzliche Abtastung infolge Entwicklung der Indikatrix der Lichtstrahlungsverteilung in Form eines Zylinders an dem Distalende des Katheters erreicht, dessen Länge mit den Abmessungen eines jeweiligen, pathologischen Herdes übereinstimmt.

Kurzbeschreibung der Erfindung

Nachstehend wird die Erfindung anhand der Beschreibung des konkreten Ausführungsbeispiels und der angelegten Zeichnung näher erläutert, in der der erfindungsgemässe biegsame durchsichtige Katheter zur Lichtbehandlung der röhrenförmigen Hohlräume des Organismus in Gesamtansicht teilweise geschnitten wiedergegeben ist.

Beste Ausführungsvariante der Erfindung

Der biegsame durchsichtige Katheter zur Lichtbehandlung der röhrenförmigen Hohlräume des Organismus enthält erfindungsgemäss eine Umhüllung I mit ihrem geschlossenen Distalende 2. Innerhalb dieser Umhüllung I ist ein Lichtleiter 3 mit einem Abstand von dem geschlossenen Distalende 2 und der Umhüllung I koaxial hindurchgezogen, zwischen dessen Stirnflanke 4 und dem geschlossenen Distalende 2 sich ein Hohlraum herausbildet, in welchem der durchsichtige, lichtstreuende, plastische Stoff 5 untergebracht ist.

Der Abstand zwischen dem geschlossenen Distalende 2 und der Stirnflanke 4 des Lichtleiters 3 ist jeweils mit der Streckenlänge des jeweiligen, pathologischen Herdes des innerhalb des röhrenförmigen Hohlräumes übereinstimmend gewählt.

Der erwähnte durchsichtige, lichtstreuende plasti-

- 4 -

sche Stoff 5 weist ein Brechungsverhältnis n auf, das sich aus der Beziehung

$$n \geq \sqrt{A^2 + n_I^2}$$

ergibt, worin

A die Apertur des Lichtleiters 3, $n_I$ das Brechungsverhältnis des Werkstoffs der Umhüllung I bedeuten.

Anstelle des erwähnten durchsichtigen lichtstreuenden plastischen Stoffs 5 wird eine Einstoffmischung auf Grund von Polyorganosiloxan mit dem Katalysator und Inhibitor verwendet, die das Brechungsverhältnis n = I,43 bis I,54 aufweist.

Die Umhüllung I des Katheters I ist erfindungsgemäss aus Polyvinylchlorid oder Silikon mit dem Brechungsverhältnis $n_I$ = I,42 bis I,52 hergestellt.

Der Lichtleiter 3 weist einen Durchmesser von 300 $\mu$m bis 2,5 mm auf. Die Apertur A des Lichtleiters 3 erreicht 0,I56. Mit den Werten von der Apertur des Lichtleiters 3 = 0,I56, $n_I$ = I,42 und $n_2$ = I,52 wird n = I,43 bzw. n = I,54 gewählt.

Die Arbeitsweise des erfindungsgemässen durchsichtigen Katheters zur Lichtbehandlung der röhrenförmigen Hohlräume des Organismus besteht im folgenden.

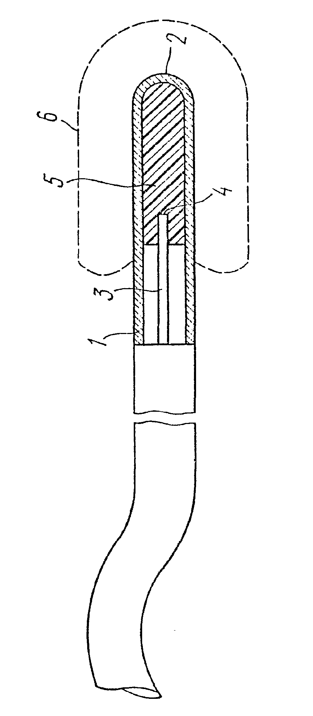

Der erfindungsgemässe Katheter wird zunächst in den jeweiligen Hohlraum des Organismus eingeführt. Die Laserstrahlung wird dann über den Lichtleiter 3 (aus der Zeichnung nicht ersichtlich) übertragen.

Ein durch die Stirnflanke 4 des Lichtleiters 3 hindurchtretender Strahlungsteil wird innerhalb des Plaststoffes 5 zerstreut, während der andere Strahlungsteil in Übereinstimmung mit dem Gesetzt der inneren Gesamtspiegelung von den Wänden der Umhüllung I des Katheters, erfindungsgemäss, unter Bewirkung eines Aufleuchtens des langgestreckten Abschnitts der Umhüllung I des erfindungsgemässen Katheters reflektiert wird. Die dabei einsetzende Indikatrix der Lichtstrahlungsverteilung erhält die Gestalt eines Zylinders, der die Gleichmässigkeit der Belichtung des langgestreckten pathologischen Herdes des jeweiligen röhrenförmigen Hohlraumes unter

- 5 -

einer wesentlichen Steigerung der Lichtbehandlungswirksamkeit der röhrenförmigen Hohlräume erreicht wird, da hierbei die Laserstrahlung an dem ganzen pathologischen Herd während der Durchführung des jeweiligen Heilverfahrens angreift.

Industrielle Anwendbarkeit

Der biegsame durchsichtige Katheter zur Lichtbehandlung der röhrenförmigen Hohlräume des Organismus ermöglicht erfindungsgemäss einen unmittelbaren Eingriff der Laserstrahlung an den pathologischen Herden innerhalb der röhrenförmigen Hohlräume des Organismus, beispielsweise bei der Lichtbehandlung der Brandwunden innerhalb der Speiseröhre des Organismus mit einer Länge von höchstens 24 cm und der Fissuren des Mastdarms unterschiedlicher Langstreckung zu erreichen.

- 6 -

PATENTANSPRUCH:

Biegsamer durchsichtiger Katheter zur Lichtbehandlung der röhrenförmigen Hohlräume von Organismus mit seinem geschlossenen Distalende (2), in einem gewissen Abstand von welchem innerhalb der Umhüllung (I), des Katheters mit dieser koaxial ein Lichtleiter (3) hindurchgezogen ist, zwischen dessen Stirnflanke (4) und dem geschlossenen Distalende (2) sich ein Hohlraum herausbildet, dadurch g e k e n n z e i c h.n e t, dass in diesem Katheter ein durchsichtiger lichtstreuender plastischer Stoff (5) mit seinem Brechungsverhältnis (n) angewandt wird, das sich aus der Beziehung

$$n \geqq \sqrt{A^2 + n_I^{\,2}}$$

ergibt, in der

A die Apertur des Lichtleiters (3),
$n_I$ das Brechungsverhältnis des Werkstoffs der Umhüllung (I) bedeuten,
welcher Plaststoff innerhalb des Hohlraums zwischen dem geschlossenen Distalende (2) und der Stirnflanke (4) des Lichtleiters (3) untergebracht ist, der Abstand zwischen denen mit der jeweiligen Streckenlänge des pathologischen Herdes des röhrenförmigen Hohlraums übereinstimmt.

## I. CLASSIFICATION OF SUBJECT MATTER (If several classification symbols apply, indicate all)

According to International Patent Classification (IPC) or to both National Classification and IPC

IPC4-A 61 N 5/06

## II. FIELDS SEARCHED

Minimum Documentation Searched

| Classification System | Classification Symbols |
|---|---|
| IPC4 | A 61 N 5/00, 5/06 |

Documentation Searched other than Minimum Documentation
to the Extent that such Documents are Included in the Fields Searched

## III. DOCUMENTS CONSIDERED TO BE RELEVANT

| Category | Citation of Document, with indication, where appropriate, of the relevant passages | Relevant to Claim No. |
|---|---|---|
| A | DE,AI,3532604(OLYMPUS OPTICAL CO.Ltd.), 27 MARCH 1986,see fig.1,the abstract    & JP,A2,61-071039,II.04.86 US,A,4676231, 30.06.87 | I |
| A | US,A,4248214,(ROBERT S. KISH) 3 February 1981 see the abstract fig.7(cited in the description) | I |
| A | Lasers in Surgery and Medicine,N6,1986, (Alan R. Liss,Inc.),M.Arnfield et al. "Cylindrical Irradiator Fiber Tip for Photodinamic Therapy",p. 150, 151 | I |
| A | SU,A1,1118371, (Volgogradsky Gosudarstvenny Meditsinsky Institut), 15 OCTOBER 1984, see column 1,lines 42-45;column 2, lines-1-4,the drawing. | I |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 20 JUNE 1989 (20.06.89) | 19 JULY 1989 (19.07.89) |
| International Searching Authority | Signature of Authorized Officer |
| ISA/SU | |

Form PCT/ISA/210 (second sheet) (January 1985)